# EUROPEAN PATENT APPLICATION

(11) **EP 4 212 189 A1**
(43) Date of publication of application: **19.07.2023**
(21) Application number: 22305039.4
(22) Date of filing: 17.01.2022
(51) Int. Cl.: A61L 9/012, A61L 9/014, A61L 9/12, A24B 15/00

(54) **VOLATILE SUBSTANCE DEVICE, USES AND METHODS RELATING TO SAME**

(71) Applicant: V. Mane Fils, 06620 Le Bar-sur-Loup (FR)
(72) Inventor: HANNETEL, Jean-Michel, 06130 Grasse (FR); TARDIEU, Audrey, 06130 Grasse (FR)
(74) Representative: Murgitroyd & Company

(57) **Abstract**

A fragrance delivery device and uses thereof are provided. The device includes a plurality of fragrance-loaded capsules; a biodegradable absorbent medium positioned adjacent the fragrance-loaded capsules along an axial direction in both directions; and an outer paper covering surrounding and containing the fragrance-loaded capsules and the biodegradable absorbent medium. Upon squeezing the capsule, the shell is ruptured releasing the liquid core, which is absorbed by the adjacent absorbent medium thereby gradually diffusing the fragrance compound toward the outer paper covering and then diffusing into the adjacent surroundings. The outer paper covering contains printed indicia including a two-dimensional barcode, which connects the user to a remote server. Upon detection, decoding, and/or transmission of the two dimensional bar code by a visual detection application residing on a portable electronic device to the remote server, information relating to the fragrance delivery device is transmitted to and displayed on the portable electronic device.

## Description

### FIELD OF THE INVENTION

The present invention relates generally to a volatile substance device, and more particularly to a fragrance delivery device, uses, and methods for enhancing products and services related thereto.

### BACKGROUND OF THE INVENTION

Volatile fragrance manufacturers have long sought to make sample fragrances available to its customers and/or consumers in order to entice a purchase. Volatile fragrances, such as consumer fine fragrances, are advertised in multiple types of media and are generally sold at a point of sale counter in a retail store. Although brand name recognition is important to fragrance consumers, so also is the actual scent of the fragrance. More often than not, a fragrance consumer will want to sample any new and/or unfamiliar fragrance prior to purchasing a quantity of it.

One type of device used to economically dispense samples of fragrance is the sample atomizer. The atomizers are used at a point of sale display to spray a measured amount of the perfume into the air or onto the customer's hand or wrist. One disadvantage of the atomizer is that if it is sprayed into the air it is hard to get a good sample of the scent. However, if it is sprayed on the customer's hand or wrist, the number of different scent samples, which can be made is effectively limited to two, one for each hand or wrist.

Of course, unscented paper card stock or blotters, such as those used by perfume scientist, may serve as a substrate to which a volatile fragrance is applied and absorbed. The volatile fragrance emanating from the sprayed blotter may then be smelled by the user. However, as the volatile fragrance slowly evaporates, so does the intensity of the scent and thus, its useful duration. Moreover, when sampling multiple fragrances, it is easy to forget which volatile fragrance is applied to which blotter, unless the user manually records information on the blotter or applies a label thereto bearing details about each volatile fragrance.

Other common devices for sampling fragrances include the so-called "peel and sniff" or "scratch and sniff' cards, such as those described in WO92/14607A1 or US2015/0140054A1. These fragrance-containing sample cards are often times distributed in magazines or mailers. The basic idea behind peel-and-sniff or scratch-and-sniff cards is to bond an absorbed fragrance (e.g., on talc) or encapsulated volatile fragrance (e.g., gelatin or plastic microspheres) to the card surface (e.g., using a lacquer or adhesive). Typically, sampling cards have a reversibly removable, impervious layer covering the perfumed region. When the perfumed region of the card is uncovered, scratched, and/or pressed, the volatilized fragrance may be exposed and smelled. While these cards are often printed with indicia relating to the details about the fragrance, e.g., brand name, manufacture, composition, etc., the adhesives used in this technology often interfere with the accurate reproduction of the "true" scent of the perfume product and/or may degrade over time.

Of course, other types of fragrance delivery devices are known. For example, WO2017017387 A1, assigned to SAS Carestia, describes a rectangular plate having two outer walls sandwiching an inner plate and one or more generic capsules. The inner plate is made in the form of open-cell foam plastic, in the form of a nonwoven, or with a structure having the effect of delaying the time taken by the volatile substance to migrate from the capsule once broken to the permeable outer wall. While the outer wall is reported to be permeable to the fragrance, the reported construction material is paper or cardboard with a grammage in the range of common business cards or invitation cards. The capsule details (e.g., shell, fragrance, solvent(s), texture characteristics, etc.) are not described.

Another odorizer or freshener article described in WO9844961 A1, assigned to R.J. Reynolds Tobacco Company, involves embedding or injecting a fragrance, in a liquid or powder form or in a breakable fragrance-containing capsule, in a web or filamentary tow material, and may further include an antistatic additive for use in clothes dryer. Again, capsule details (e.g., shell, fragrance, solvent(s), texture characteristics, etc.) are not described.

Accordingly, new devices and methods are needed for sampling and/or marketing volatile fragrances that overcome one or more of the deficiencies of the traditional approaches.

### SUMMARY OF THE INVENTION

Certain aspects of the present disclosure are described in the appended claims. There are additional features and advantages of the subject matter described herein. They will become apparent as this specification proceeds. The various features described in the claims and below for various embodiments may be used in combination or separately. For example, specified ranges may be inclusive of their recited endpoints, unless explicitly excluded. Any particular embodiment need not provide all features noted above, nor solve all problems or address all issues noted above.

According to embodiments of the present invention, a fragrance delivery device is provided that comprises, consists essentially of, or consists of, a plurality of fragrance-loaded capsules; an absorbent medium positioned adjacent the fragrance-loaded capsules along an axial direction in both directions; and an outer paper covering surrounding and containing the fragrance-loaded capsules and the absorbent medium. The outer paper covering allows the fragrance compound to diffuse therethrough and comprises printed indicia, for example including a one- or two-dimensional barcode, or consisting of a one-or two-dimensional barcode, visible to the user. The fragrance-loaded capsules are seamless capsules comprising (consisting essentially of, or consisting of) a capsule shell surrounding a liquid core containing a fragrance compound and a non-glyceride solvent, and the liquid core has a relative density between 0.85 and 0.99. The capsule shell of the seamless capsules comprises a hydrocolloid mixture, and the shell is breakable having an initial crush strength (Cᵢ) from 0.5 to 2.5 kg, elasticity up to 60%, and a shape ratio equal to 0.9 or higher. Upon rupturing the capsule shell by application of a sufficient crushing force, the adjacent absorbent medium absorbs the liquid core thereby gradually transporting the fragrance compounds toward the outer paper covering and then diffusing into the adjacent surroundings. Surprisingly, it has been discovered that the fragrance delivery device described herein, containing the fragrance-loaded capsules, provides the user a long lasting sensory experience with better fidelity of the top, middle, and bottom notes versus a traditional blotter.

According to an embodiment of the present invention, the fragrance delivery device is combined with a remote server to further provide fragrance delivery system for allowing a user to utilize a portable electronic device to retrieve information relating to the fragrance-loaded capsule in response to the portable electronic device detecting printed indicia, e.g., 1D or 2D barcode, on the surface of the outer paper covering. The fragrance delivery system may also allow a two-way exchange of information, whereby the end user may register an account and provide feedback on the fragrance delivery device.

### BRIEF DESCRIPTION OF THE DRAWINGS

The accompanying drawings, which are incorporated in and constitute a part of this specification, illustrate embodiments of the invention and, together with a general description of the invention given above, and the detailed description given below, serve to explain the invention. It will be appreciated that for purposes of clarity and where deemed appropriate, reference numerals have been repeated in the figures to indicate corresponding features.
FIG. 1 is a plan view of a fragrance delivery device, in accordance with an embodiment of the present invention;
FIG. 2 is a cross sectional view of the fragrance delivery device depicted in FIG. 1 taken along dashed line 1A - 1A, in accordance with a first embodiment;
FIG. 3 is a cross sectional view of the fragrance delivery device depicted in FIG. 1 taken along dashed line 1A - 1A, in accordance with a second embodiment;
FIG. 4 is a schematic showing a fragrance delivery device, in accordance with another embodiment of the present invention;
FIG. 5 is a chart showing comparative testing of a conventional perfumeimpregnated blotter versus the inventive fragrance delivery device rating the overall fragrance intensity; and
FIG. 6 is a chart showing comparative testing of a conventional perfumeimpregnated blotter versus the inventive fragrance delivery device rating the intensities of the top, heart, and bottom notes of the fragrance.

### DETAILED DESCRIPTION

Unless otherwise explained, all technical and scientific terms used herein have the same meaning as commonly understood by one of ordinary skill in the art to which this disclosure belongs. In case of conflict, the present specification, including explanations of terms, will control. The singular terms "a," "an," "at least one," and "the" include plural referents unless context clearly indicates otherwise. Similarly, the word "or" is intended to include "and" unless the context clearly indicates otherwise. The term "comprising" means "including;" hence, "comprising A or B" means including A or B, as well as A and B together.

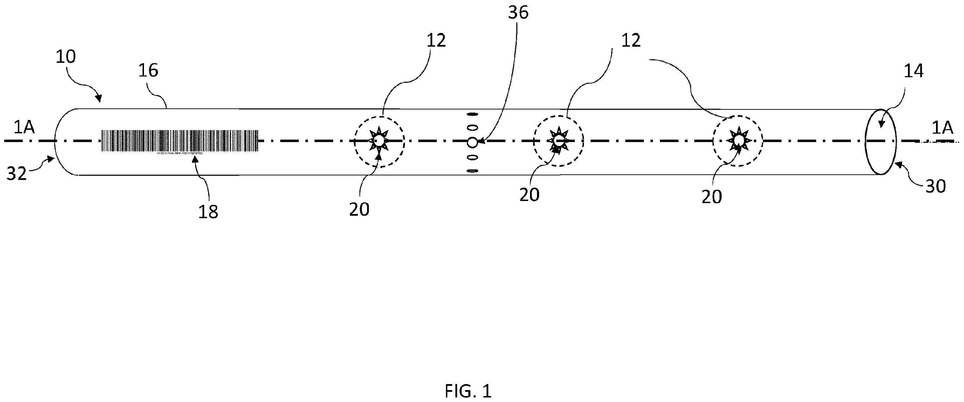

FIG. 1 is a plan view of a fragrance delivery device 10, which comprises a plurality of fragrance-loaded capsules **12** (shown in dashed outline) and an outer paper covering **16** surrounding and containing the fragrance-loaded capsules **12.** The outer paper covering **16** comprises a printed indicia **18** and capsule location markings **20** visible to the observer. These visible features enable the observer to obtain information relating to the fragrance-loaded capsule **12,** as well as each capsule location under the outer paper covering **16,** as explained further herein.

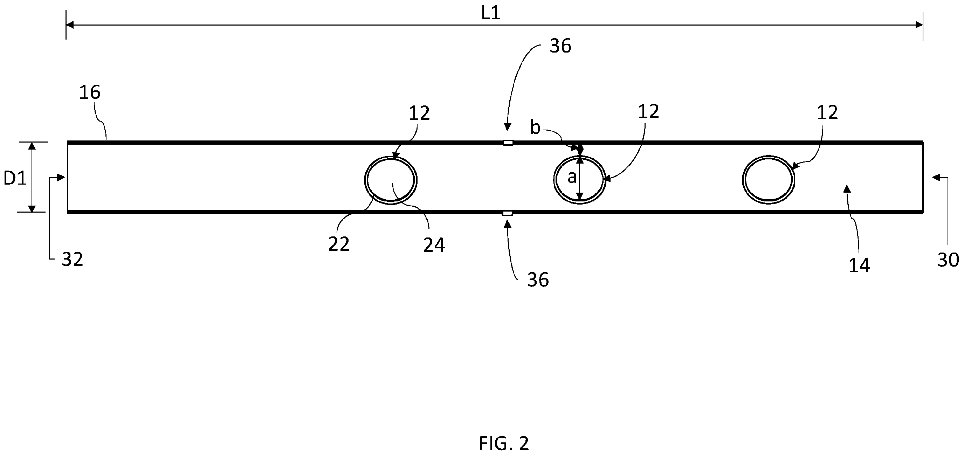

FIG. 2 is a cross sectional view of the fragrance delivery device depicted in FIG. 1 taken along dashed line 1A - 1A, and shows a first embodiment where an absorbent medium **14** is positioned adjacent the fragrance-loaded capsule **12** along an axial direction in both directions in a continuous configuration.

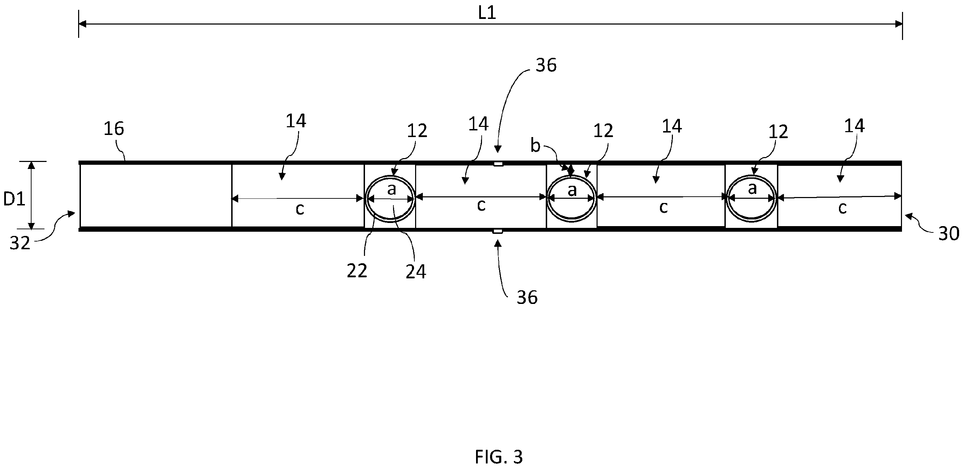

FIG. 3 is a cross sectional view of the fragrance delivery device depicted in FIG. 1 taken along dashed line 1A - 1A, and shows a second embodiment where individual portions of absorbent medium **14** having a length c are positioned adjacent the fragrance-loaded capsule **12** along an axial direction in both directions.

In FIGS. 1-3, the fragrance-loaded capsule **12** comprises a capsule shell **22** surrounding a liquid core **24** containing a fragrance compound and optionally a solvent.

In accordance with embodiments of the present invention, the term "capsule" designates a core-shell structure, where the capsule shell **22** surrounds a liquid core **24,** and is distinguished from a matrix system where little droplets of liquid are dispersed in a continuous matrix of a shell-forming material. In accordance with embodiments of the present invention, the fragrance-loaded capsule **12** is seamless, which obviates having to weld together two half-shells, as is the case with so-called "soft-gel" capsules. Thus, the seamless fragrance-loaded capsule **12** has the advantage of avoiding leaks associated with rupture of a weak weld portion.

In accordance with embodiments of the present invention, the term "breakable capsule" refers to a capsule as defined above, in which the shell can be broken by applying force to opposing sides of the outer surface of the capsule. Upon rupturing the capsule shell **22** by application of a sufficient crushing force, the liquid core **24** is absorbed by the adjacent absorbent medium **14** thereby gradually diffusing the fragrance compound toward the outer paper covering **16** and then diffusing into the adjacent surroundings. The breakable capsules according to the invention have a hardness (initial crushing strength (Cᵢ)) between 0.5 and 2.5 kg, and thus is breakable by application of force between two fingers, and makes an audible noise when broken.

The capsule shell **22** according to the invention advantageously comprises at least one hydrocolloid gelling agent. Preferably, the hydrocolloid gelling agent is a biobased polymer. By "bio-based," it is meant a synthetic polymer that is at least partially (generally > 20%) or completely obtained from biomass or derivatives thereof. The biobased nature of a polymer can in particular be determined from its C14 content, according to standard ASTM D6866-21.

The hydrocolloid gelling agent of the capsule shell **22** of the fragrance-loaded capsule **12** is preferably selected from the group consisting of gellan gum, gelatin (of animal or biotechnological origin), collagen, alginates, carrageenans, agar-agar, chitosan and its derivatives, pectins, gum arabic, ghatti gum, pullulan gum, mannan gum, starch and derivatives of starch, vegetable protein, or modified cellulose, and combinations thereof. To prepare an animal-free seamless capsule, the shell material of the breakable capsule would be void of any gelatin derived from animal sources.

In a preferred embodiment, the hydrocolloid gelling agent comprises gellan gum, used alone or in combination with gelatin. In another preferred embodiment, the hydrocolloid gelling agent comprises carrageenans. Based on a total mass of the dry weight ingredients in the shell, the hydrocolloid gelling agent(s) is present in an amount in the range from about 10 wt% to about 95 wt%, preferably 15 wt% to 75 wt%, and more preferably 20 wt% to 50 wt%.

The shell material further comprises a filler material. Exemplary filler materials include but are not limited to starch derivatives such as dextrin, maltodextrin, innulin, sucrose, allulose, tagatose, cyclodextrin (alpha, beta, gamma, or modified cyclodexrin); cellulose derivatives such as microcrystalline cellulose (MCC), hydroxypropylcellulose (HPC), hydroxypropylmethylcellulose (HPMC), methylcellulose (MC), or carboxymethylcellulose (CMC); a polyvinyl alcohol; polyols with non-plasticizing properties; trehalose; erythritol; maltitol; mannitol; xylitol; sorbitol; glycerol; triacetine; a polyethylene glycol, polyalcohols with plasticizing or humectant properties; or combinations of two or more of the foregoing. Based on a total mass of the dry weight ingredients in the shell, the filler may be present in an amount in the range from about 10 wt% to about 90 wt%, preferably 15 wt% to 75 wt%, and more preferably 20 wt% to 50 wt%.

As further described below, the seamless fragrance-loaded capsules **12** are formed by co-extrusion, and thus a gellable aqueous mixture of the dry ingredients (e.g., hydrocolloid gelling agent(s), filler(s), and other additive(s)) is formed by mixing with water. A typical weight ratio of water to the non-water (dry) ingredients is in a range from 1:1 to 20:1. Preferably, the water used for the external phase is purified water, such as distilled water, deionized water, or reverse osmosis water, but processing water is viable. The gellable aqueous mixture may further comprise crosslinking agents, coloring agents, sequestering agents, and the like.

The liquid core **24** comprises at least one perfuming agent/fragrance compound and at least one non-glyceride lipophilic solvent. Advantageously, the non-glyceride solvent is miscible with ethanol and has the following characteristics: a) a viscosity of less than 10 mPa•sec, as measured at a temperature of 25 °C and a shear rate of 10 s⁻¹; b) a spread value greater than 850 mm² / 10 min; and c) a relative density of between 0.85 and 0.99.

An important technical advantage of the present invention is that the non-glyceride lipophilic solvent enables the encapsulation of the liquid core using co-extrusion techniques to obtain a breakable, yet stable capsule. Moreover, the non-glyceride lipophilic solvent also provides good transport of the perfume composition (via absorption into the adjacent absorbent medium **14**) to the outer paper covering **16.**

Indeed, it is known to those skilled in the art of perfumery, that in a conventional perfume composition, it is the presence of a large quantity of ethanol that allows the evaporation of the perfume by "carrying" the molecules of perfume, and therefore the scent effect. However, co-extrusion encapsulation of a liquid core composition comprising a large amount of ethanol is impossible to perform, because ethanol is hydrophilic and de-stabilizes the interface between the liquid core and the aqueous shell-forming composition. While simply replacing ethanol with a conventional lipophilic co-extrusion solvent, such as medium chain triglycerides (MCTs), would likely enable successful co-extrusion, MCTs greatly limit the diffusion of perfume molecules, as well as can persist with a greasy feeling on the skin or leave oily marks on clothing.

Advantageously, the non-glyceride lipophilic solvents are miscible with both ethanol and the perfuming agents, and further possess no odor that interferes with the smell of the perfume composition. As used herein, the term "miscible" is understood to mean that the non-glyceride lipophilic solvent can be mixed with perfume materials and with ethanol, forming a homogeneous mixture. In some instances, depending on the extent of lipophilicity of the perfume materials, it may be advantageous to use a small quantity of ethanol (e.g., between 0 to 10 wt%) in the liquid core composition during the co-extrusion process, the majority of which will evaporate during the drying of the capsules. Similarly, a small quantity of a glyceride solvent is also permissible. However, the non-glyceride lipophilic solvent used in embodiments of the present invention is the major solvent, i.e., greater than 50% by mass.

Thus, the non-glyceride hydrophilic solvent, as defined according to the present invention, provides the following advantages to the liquid core **24:** a) stable liquid core formulation; b) good carrier properties to transport the perfuming agent(s) through the absorbent medium **14** to the outer paper covering **16;** c) good evaporation profile of the perfume composition through the outer paper covering **16;** d) non-greasy or oily feeling; and e) does not stain clothing materials.

In an embodiment, the non-glyceride lipophilic solvent is selected from the group consisting of non-glycerol esters (e.g., isopropyl myristate, isoadipate, and/or cococaprylate); silicones (e.g., polydimethylsiloxanes, octamethyltrisiloxane, and/or dimethicones), vegetable oils (e.g., grapeseed oil), and combinations thereof. In an embodiment, vegetable oils are preferred in order to obtain a capsule that is bio-based and biodegradable. In another embodiment, the non-glyceride lipophilic solvent is selected from the group consisting of isopropyl myristate, isoadipate, cococaprylate, polydimethylsiloxane, octamethyltrisiloxane, dimethicone, and grapeseed oil, and combinations thereof.

The relative density of the non-glyceride lipophilic solvent is preferably between 0.85 and 0.99. By definition, the relative density of a composition corresponds to a ratio of the density of said composition measured at 20 °C to the density of water at 4 °C. This relative density range of the non-glyceride lipophilic solvent ensures good results during the manufacture of the capsules by the so-called co-extrusion process. Advantageously, the relative density of the total composition of the liquid core (e.g., perfuming agent(s); lipophilic solvent(s), ethanol, if present) is between 0.85 and 0.99.

Advantageously, the non-glyceride lipophilic solvent should have a viscosity of less than 10 mPa•sec, as measured at a temperature of 25 °C and a shear rate of 10 s⁻¹. The viscosity (η) measurements, expressed in mPa•sec, are performed using a Haake MARS III rheometer, at 25 °C using a 5 mm cone with a 2-degree angle, and a shear rate of 10 sec⁻¹.

The non-glyceride lipophilic solvent should have a spread value greater than 850 mm² / 10 min. The spread value (or spreadability) of any substance may be generally defined as the ability of said substance to cover a surface within a defined period. Advantageously, the spread value of a lipophilic solvent (chosen from esters, silicones, vegetable and mineral oils) can be calculated for a 10 minute interval using equations taken from the literature (M. Douguet and al., "Spreading properties of cosmetic emollients: Use of synthetic skin surface to elucidate structural effects." Colloids Surf B Biointerfaces, 154 (2017) 307-314). Indeed, following statistical analyzes by partial least squares regression, it was found that the viscosity measured at 25 °C (x) is the most reliable variable to predict the spread value (*y*), as a function of the nature of the non-glyceride lipophilic solvent, according to various chemospecific logarithmic regressions: For esters: *y* = -255 x *log(x)* + 1315; for silicones: *y* = -222 x *log(x)* + 1670; and for vegetable oils: *y* = -101 x *log*(*x*) + 748. These equations may be used in the screening or selection of non-glyceride lipophilic solvents.

In accordance with embodiments of the present invention, the non-glyceride lipophilic solvent is between 10 wt% and 99.99 wt%, based on the total weight of the liquid core composition. A small quantity of ethanol (e.g., between 0 to 10 wt%, based on the total weight of the liquid core composition) and/or a small quantity of a glyceride solvent (e.g., between 0 to 5 wt%, based on the total weight of the liquid core composition) may be used with the non-glyceride lipophilic solvent. For example, the non-glyceride lipophilic solvent may be present in the liquid core between 15 wt% and 90 wt%, or between 20 wt% and 80 wt%, or between 25 wt% and 75 wt%, based on the total weight of the liquid core composition.

Exemplary perfuming agents/fragrance compounds useful for the liquid core **24** in the fragrance-loaded capsules include, but are not limited to, one or more aromatic or fragrance molecules as conventionally used in the formulation of flavoring or fragrance compositions. For example, fragrance molecules include aromatic, terpenic and/or sesquiterpenic hydrocarbons, and more particularly essential oils, alcohols, aldehydes, ketones, phenols, carboxylic acids in their various forms, aromatic acetals and ethers, nitrogenous heterocycles, sulfides, disulfides and mercaptans which may be aromatic or non aromatic. Such odorous substances are mentioned, for example, in S. Arctander, "Perfume and Flavor Chemicals" Vol. I and II, (Montclair, NJ, 1969), or also in "Common Fragrance and Flavor Materials", 5th Ed., Wiley-VCH, Weinheim, 2006. The perfuming agents may also include one or more captive agents, including but not limited to, Orcanox^{™} 1,5,5,9-tetramethyl-13-oxatricyclo[8.3.0.0] tridecane; Betahydrane^{™} (3-benzyl-tetrahydropyran); Antillone^{™} (9-decen-2-one); Noreenal^{™} ((±)-6,8-Dimethylnon-7-enal); and/or Pescagreen^{™} (2-(2,4,4-trimethyl- cyclopentyl)-acrylonitrile). In an embodiment, the perfuming agent(s) in the liquid core is between 0.01 wt% to 90 wt%, based on the total weight of liquid core composition. For example, the perfuming agent(s) may be present in the liquid core in an amount between 1 wt% and 85 wt%, or between 5 wt% and 80 wt%, or between 10 wt% and 75 wt%, based on the total weight of the liquid core composition.

In accordance with an embodiment, the amount of the perfuming agent(s) in the liquid core may be defined based on the end-use application, e.g., aroma therapy, fragrance sampling, air freshening, etc. Furthermore, the diameter of the dried fragrance-loaded capsule **12** and/or the thickness of the capsule shell may be adjusted to achieve a desired volume of the liquid core for a specific end-use application.

Advantageously, the perfuming agents and other ingredients can be evaluated for their environmental impact using MANE's GREEN MOTION^{™} index. This index assesses the health, safety, and environment impacts of manufactured ingredients for the Flavor and Fragrance industry on a 0 to 100 scale. The safer and the less impactful the process, the higher the rating. The GREEN MOTION^{™} index is fundamentally tied to the 12 green chemistry principles, as introduced by Anastas and Warner (Green Chemistry, Theory and Practice, Oxford University Press, New-York, 1998). However, GREEN MOTION^{™} focuses on 7 fundamental concepts, i.e., raw material, solvent, hazard and toxicity of the reagents, reaction, process, hazard and toxicity of the final product and waste.

In addition to perfuming agents and the non-glyceride lipophilic solvent, the liquid core **24** of the fragrance-loaded capsule **12** can advantageously comprise a fixing agent in the preparation formulation in order to inhibit the rapid volatilization of the perfuming agent(s) and thus provide a longer lasting perfume. Fixing agents are less volatile than the perfuming agents themselves, and thus reduce the evaporation rate of the fragrant components. Non-limiting types of suitable fixing agents include absolutes; concretes; resins; derivatives of glucose, sucrose, sorbitol, citric acid, and salicylic acid; celluloses (especially ethylcellulose); seaweed extracts; triglycerides; oils such as castor oil and its derivatives; emollients such as certain esters such as ethyl hexyl glycerin; polyurethanes; polyamides; certain silicones; liquid paraffin; glycol ethers; pyrogenic silica; and quaternary ammonium salts.

According to a preferred embodiment of the invention, the liquid core **24** of the fragrance-loaded capsule **12** represents 50% to 95% (w/w) of said capsule, preferably 80% to 92%, more preferably 85% to 92%. Conversely, in this preferred embodiment, the capsule shell **22** of the fragrance-loaded capsule **12** represents 5% to 50% (w/w) of said capsule, preferably 8% to 20%, more preferably 8% to 15%.

The seamless fragrance-loaded capsules **12** can be manufactured by a co-extrusion process, as described in patent EP513603. A general procedure for preparing seamless capsules is described below. The outer phase of a gellable aqueous mixture of the dry ingredients (e.g., hydrocolloid gelling agent(s), filler(s), and other additive(s)) and the inner oily phase of the liquid core are pumped individually through a submerged coaxial nozzle assembly to form a concentric composite flow, which separates into discrete concentric drops under the effect of applied vibrational energy. The discharge from the coaxial nozzle is submerged in a carrier fluid (eg, medium chain triglyceride (MCT)), which is at a temperature below the gel temperature of the gellable aqueous mixture. The gellable aqueous mixture is thus cooled and forms the hydrated hydrocolloid-containing shell part of the capsule. The capsules thus formed are then collected and centrifuged to remove a majority of the residual MCT. The centrifuged capsules may be further treated with a dessicant or drying agent (e.g., starch or silica), then dried with air between 30 and 45 °C. The resulting dried capsules are then collected and sieved. Dried fragrance-loaded capsules **12,** prepared according to embodiments of the present invention, have a homogeneous and smooth appearance, and are spherical or substantially spherical (as measured by the average ratio of the width to the length of the microcapsules). In a preferred embodiment, the dried seamless fragrance-loaded capsules **12** possess texture properties (e.g., initial crush strength or elasticity) to withstand further manipulation and incorporation into the fragrance delivery device, yet may be broken by the force of squeezing between two fingers (accompanied by an audible pop upon breakage).

Advantageously, the outer diameter of the dried fragrance-loaded capsule **12** is between 2 and 10 mm, preferably 3 to 5 mm, more preferably 3.4 to 4.8, and even more preferably 3.5 to 4.5 mm; the thickness of the shell **22** of the fragrance-loaded capsule **12** is 10 to 500 microns, preferably 30 to 150 microns, more preferably 50 to 80 microns; and the ratio of the capsule diameter / shell thickness is in the range 10 to 100, preferably 50 to 70. The dried, fragrance-loaded seamless capsules **12** have an initial crush strength (Cᵢ) from 0.5 to 2.5 kg, elasticity up to 60%, and a shape ratio equal to 0.9 or higher. For determining the foregoing characteristics, the moisture content (measured by Karl Fisher titration) of the dried capsule is less than 5 wt%, based on the entire weight of the capsule.

The initial crush strength (Cᵢ) (also called hardness or force at break) of the capsules is measured using a TA.XTplus texture analyzer from Stable Micro System Ltd. (Surrey, UK) in compression mode with a 5 Kg load cell; Probe: P0.5 - ½ diameter DELRIN^{®} cylinder; cylinder speed 0.5 mm/sec; resolution of 0.01 Kg, taking an average on 20 capsules. The capsule is positioned on the TA.XT plus device between the base and the probe. Vertical compressive force is then continuously applied onto one particle until the breakable shell ruptures and simultaneously the built-in gauge records force (in kilograms (Kg) and position (in millimeter (mm)). The breakage of the capsule is accompanied by an audible noise verifying its rupture and release of the liquid core.

"Deformation" is a ratio of the distance at break and the initial capsule size, where the "distance at break" (in mm) is the distance covered by the probe from the contact of the capsule until the capsule's breaking point, as measured using the TA.XTplus texture analyzer described above. "Elasticity" is the deformation value expressed in percent.

The shape ratio (or symmetry) of the dried capsules is 0.9 or greater. Equivalent diameter and symmetry/ shape ratio are measured by CPA 2-1 from Haver & Boecker. The CPA is photo-optical unit for measuring size and shape analysis of dry particles (e.g. capsules). CPA unit consists in a feeding unit, an optical sensor, a camera, a special light source and an electronic module. The capsules fall in front of the camera. The shadow projections are detected by the camera and evaluate with CPA Serv software in real time.

The fragrance-loaded capsules **12** are embedded in an absorbent medium **14** positioned along an axial direction and surrounded and retained by the outer paper covering **16.** The diffusivity of the fragrance may be enhanced by modification of specific characteristics of the absorbent medium **14** (such as chemical composition, density, pressure drop, hardness, form, and diameter) and/or by modification of specific characteristics of the outer paper covering **16** (such as coating, perforations, grammage, and porosity).

As shown in FIG. 2, the absorbent medium **14** may have a continuous configuration with cavities to accommodate the substantially spherical or spherical fragrance-loaded capsule **12.** Alternatively, the absorbent medium **14** may be individual portions, as shown in FIG. 3. Although not shown, the absorbent medium **14** may also be configured with radially extending or axially extending channels to enhance the absorption and/or diffusion rate of the fragrance.

In accordance with an embodiment of the present invention, the absorbent medium **14** comprises a biodegradable / biocompostable polymer. As used herein, a "biodegradable" matter means a matter capable of decomposing rapidly under natural conditions within one year using the standard test method (ISO 14855-2:2018), and biodegradation is the process by which organic substances are decomposed by microorganisms (mainly aerobic bacteria) into simpler substances such as carbon dioxide, water and ammonia.

Traditional cigarette filters are made of cellulose acetate, which is a chemicallymodified natural polymer. While acetylation of hydroxyl groups of cellulose imparts favorable and useful properties, this hydrophobated form also impedes its ability to biodegrade. Thus, cellulose acetate is not considered to be biodegradable, unless it is combined with other ingredients or materials which facilitate deactylation, such as a "biodegradation promoting agent", as described in WO0053832A1, or by materials that catalyze the hydrolysis of the cellulose acetate filament, as described in WO2009079202A1.

Non-limiting examples of biodegradable, absorbent medium materials include cellulose, flax, cotton, manila hemp, polylactic acid, or polylactide, as well as biodegradable polyesters (e.g., polyglycolic acid, polylactic acid, polyhydroxyalkanoates, polycaprolactone, polybutylene succinate adipate and copolymers or blends thereof), as described in WO2013019616A2, assigned to RJ Reynolds, which is incorporated herein by reference in its entirety. Non-limiting examples of commercially-available biobased and biodegradable absorbent medium materials include Eastman ESTRON^{™} (Kingsport, TN); biodegradable tow produced by Tianjin NAT Technology (Tianjin, China); Greenbutts^{™} produced by Greenbutts LLC (San Diego, CA); Cerdia^{®} DE-Tow produced by Cerdia International GMBH (Basel, Switzerland); or ECO Active^{™} produced by Essentra Filter Products Development Co. Pte. Ltd (Singapore).

In an embodiment, the absorbent medium **14** material may comprise randomly oriented regenerated cellulose fibers and cellulose acetate fibers, and may further comprise a binder. The term "regenerated cellulose fibers" is used herein to mean cellulose fibers which have been formed by processing a naturally occurring cellulose material to provide cellulose fibers having a desired physically property. A typical process for forming regenerated cellulose fibers includes the steps of: pulping a naturally occurring cellulose material, such as wood chips, to form a pulp; subjecting the pulp to one or more treatment steps to alter the physical properties of the cellulose; and forming fibers of regenerated cellulose from the treated pulp, for example by spinning cellulose fibers by passing the pulp through a triangular spinneret, providing a cross-section Y shape.

In accordance with an embodiment of the present invention, the absorbent medium material is characterized having a Denier filament in a range of 2.5Y to 8.0Y, and a total linear density 10000-40000. Dernier filament is weight expressed in length unit defined as the weight in grams of 9000 m of the linear cellulosic material. Dernier is a direct metric system where lower numbers are thinner dimensions and higher numbers are thicker dimensions. A single filament with a 9000 m length and with a weight of 1 gram is equal to dernier 1. Total dernier is a weight in grams of absorbent material fiber with a length of 9000 m. The absorbent medium material may be characterized by its dernier filament and total dernier. For example: a 6.0 Y 17000 absorbent material has a dernier per filament (or filament dernier) of 6.0 and a total dernier of 17000, where the cross section Y is the one commonly used in the tobacco industry. In an embodiment, the absorbent medium material has a Denier filament in a range of 4.0 to 8.0, and a total linear density in a range from 12000 to 30000.

Advantageously, using an absorbent material that comprises randomly oriented regenerated cellulose improves the degradation of the filtration material. This is because the randomly oriented fibers can more easily disperse after the fragrance deliver device **10** has been discarded, particularly when compared with the substantially continuous filaments of traditional cellulose acetate tow materials. Increased dispersion of the fibers increases the exposure of the individual fibers to the environment, thus increasing the rate at which the cellulosic material degrades.

Cellulose and its derivatives, including those used in more traditional paper applications, are preferably sourced responsibly. Exemplary environmental certifications, such as FSC (Forest Stewardship Council^{®}) or PEFC (Programme for the Endorsement of Forest Certification), are well-known certifications to ensure the traceability of the pulp source. The European standard EN 643 "European List of Standard Grades of Paper and Board for Recycling" defines paper and board materials and products, which can be recovered through recycling. According to an embodiment, the absorbent material is prepared from cellulosic material that is sourced responsibly or recycled.

According to an aspect of the present invention, the absorbent medium material is formed into a substantially cylindrical or cylindrical shape using a binder composition. One common binder material is triacetin. In an embodiment, the binder composition may further comprise one or more of dimethylisosorbide, propylene carbonate, methylbenzyl alcohol, glycerol carbonate acetate, glycerol carbonate ethyl ether, and mixtures thereof. In another embodiment, the binder composition is void of any dimethylisosorbide, propylene carbonate, methylbenzyl alcohol, glycerol and glycerol derivatives such as glycerol carbonate acetate or glycerol carbonate ethyl ether, or mixtures thereof.

The triacetin or triacetin-containing binder composition is preferably present in an amount of about 1 % to about 15% by weight of the absorbent medium, more preferably about 5% to about 13% by weight of the absorbent medium, most preferably about 8% to about 12% by weight of the absorbent medium.

The fragrance-loaded capsules **12** and the absorbent medium **14** are surrounded and retained by the outer paper covering **16.** Similar to the absorbent medium **14,** the outer paper covering **16** is also biodegradable. Suitable materials for constructing the outer paper covering include, but are not limited to cellulose and its derivatives, with or without fillers. In an embodiment, the outer paper covering **16** is nonporous. The porosity of the outer paper covering **16** may be expressed in CORESTA units (CU) and is the volumetric flow rate of air (cm³ / min) passing through a 1 cm² sample of substrate at an applied pressure difference of 1 kPa. In an embodiment, the outer paper covering has a porosity of less than 5 CU, such as 4 CU, 3 CU, 2 CU, 1 CU, or 0 CU, or in a range between any two of the foregoing.

In an embodiment, the outer paper covering **16** is coated or impregnated. The coating or impregnation can be carried out using a blade coater, a roll coater, dipping rolls, curtain coater, size press, or other conventional coating and impregnating methods. Non-limiting examples of commercially-available biobased and biodegradable outer paper coverings **16** include plug wrappers produced by Delfort Group (Traun, Austria) or plug wrappers produced by Schweitzer-Mauduit International, Inc (Alpharetta, GA).

The basis weight or grammage is a fundamental property of the outer paper covering **16.** Basis weight of paper is the weight per unit area. This can be expressed as the weight in grams per square meter (gsm or g/M²). In accordance with an embodiment, the grammage of the outer paper covering **16** can vary from 22 gsm to 90 gsm to provide good diffusivity of the fragrance, nice feeling touch of the device and rigidity to the fragrance delivery device **10,** yet inhibiting transport of solvent/oil to the user's fingers and maintaining the relative cylindrical shape of the device during usage. For example, the grammage of the outer paper covering **16** may be 22 to 90 gms, 24 to 80 gms, 26 to 70 gms, or 30 to 60 gms.

As shown in FIG. 1 and in accordance with embodiments of the present invention, the outer paper covering **16** bears printed indicia **18** and location markings **20** on its surface. While printing may be performed after assembly, it is preferable to print on the outer paper covering **16** prior to assembly while the paper is flat. Printing on a flattened outer paper covering **16,** which may be colored, allows the printing of indicia and markings with an accuracy of +/- 2 mm. The capsule location markings **20** simply and quickly identify the location of the fragrance-loaded seamless capsules.

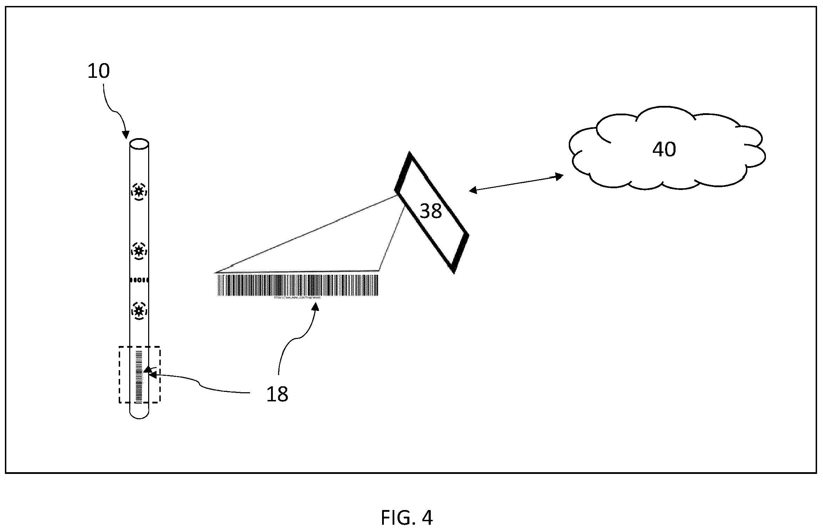

As shown in FIG. 4, the printed indicia **18** may provide information that is directly discernible by the user, such as a company name, brand name, trademark, or any other information relating to the fragrance composition within the device. Advantageously, the printed indicia **18** includes a scannable one- or two-dimensional barcode (e.g., code39, code128, QR, UPC-A, EAN-8, EAN-13, and ITF barcodes) or any other smartphone scannable code that can be photographed/detected by a portable electronic device **38** and link the consumer instantly and directly to a specific internet URL site of a remote server **40.** Accordingly, the printed indicia **18** enables the user to utilize a portable electronic device to retrieve information relating to the fragrance-loaded seamless capsule. For example, the user may have direct access to information related to the fragrance, such as its composition, ingredient origin, supplier information, GREEN MOTION^{™} index, and other details relevant to the user, such as ratings or comments from other users or perfumers.

The remote server **40** may also be configured to enable a two-way exchange of information, whereby the end user may register or create an account and provide feedback or rating on the fragrance delivery device **10** and the fragrance-loaded capsule **12** contents. Accordingly, the remote server **40** is linked to the fragrance delivery device **10** via the printed indicia **18,** whereupon detection, decoding, and/or transmission of the printed indicia **18** by a visual detection application residing on the portable electronic device **38** to the remote server **40,** information relating to the fragrance delivery device **10** is transmitted to and displayed on the portable electronic device **38.** Further, the user created file or account may be utilized to store the user's personal information, including the number and/or types of fragrance compositions sampled, as well as serve as a record for the user's likes or dislikes. The user created file may be stored on the portable electronic device **38** and/or on the remote server **40.**

Accordingly, in an embodiment of the present invention, a fragrance delivery system is provided. The system comprising, consisting essentially of, or consisting of, the fragrance delivery device **10,** and a remote server linked to the printed indicia **18,** said remote server **40** being adapted to transmit and display information relating to the fragrance delivery device **10** to/on a portable electronic device **38** upon detection, decoding, and/or transmission of printed indicia **18,** for example of the one- or two-dimensional bar code, by a visual detection application residing on the portable electronic device **38.** Furthermore, a user of the portable electronic device **38** and the fragrance delivery device **10** may be provided a file to store personal or product related information, said file being stored on the portable electronic device **38** or on the remote server **40.**

Based on the substantially spherical or spherical shape of the fragrance-loaded seamless capsules and the known art of fabricating cigarette filters with flavor-filled capsules, the fragrance delivery device of the present invention may be manufactured using conventional cigarette and capsule packaging machinery. This assembling of substantially spherical or spherical fragrance-loaded capsule **12,** absorbent medium **14,** and outer paper covering **16** may further include the use of adhesive(s) for anchoring the fragrance-loaded capsule **12** and/or absorbent medium **14** to the outer paper covering **16,** as well as gluing the seam of the outer paper covering **16.** Biodegradable adhesives suitable for anchoring the fragrance-loaded capsule **12** and/or absorbent medium **14** and gluing the seam of the outer paper covering **16** may include, for example, polyvinyl acetate (PVA) or biodegradable hot melt (HM) adhesives.

The adherence of the absorbent medium **14** to the outer paper covering **16,** along with the physical properties of the absorbent medium 14 to the outer paper covering 16 themselves, provides good structural integrity to the fragrance delivery device **10.** For example, the Borgwaldt hardness, which represents the remaining height of the fragrance delivery device's diameter relative to its original diameter after testing with a load of 2 kg for a set time of 10 seconds, may be used to characterize the fragrance delivery device **10.** The Borgwaldt hardness test is performed with a Borgwaldt DD60A Densimeter device (manufactured and made commercially available by Heinr. Borgwaldt GmbH, Germany). In accordance with embodiments of the present invention, the fragrance delivery device **10** has a Borgwaldt hardness of 50% or more, preferably 75% or more, or more preferably 80% or more.

In accordance with embodiments of the invention and in further reference to FIGS. 1-3, the fragrance delivery device **10** is configured into a cylindrical body comprising a distal end **30** and a proximal end **32,** along with a plurality of the seamless fragrance-loaded capsules **12** aligned along the axial direction of a central axis of the cylindrical body; wherein the plurality of capsules are spaced apart and separated by portions of the absorbent medium **14.** The length and diameter dimensions are not limiting with respect to the implementation of the fragrance delivery device **10.** The length (**L1**) of the cylindrical body may be in a range from about 5 cm to about 25 cm, preferably about 10 cm to about 20 cm; and the diameter (**D1**) of the cylindrical body may be in a range from about 4 mm to about 15 mm, preferably about 5 mm to about 10 mm, for example.

In an aspect, the diameter (**D1**) of the fragrance delivery device **10** is related to the diameter of the capsule (**a**), such that the diameter (**D1**) of the fragrance delivery device **10** is larger than the diameter (**a**) of the fragrance-loaded capsule **12.** As shown in FIG. 2, the fragrance-loaded capsule **12** having a diameter (**a**) is contained within the fragrance delivery device **10** having an overall diameter (**D1**), but is preferably not in contact with the outer paper covering **16,** being separated by a distance (b), which minimizes damage to the fragrance-loaded capsule **12** during the assembly of the fragrance delivery device **10.**

Additional modifications and features may be made to enhance the sensory experience from using the fragrance delivery device described herein. For example, as shown in the embodiment presented in FIG. 1, small perforations **36** may be strategically placed in the outer paper covering **16** to enhance diffusion of the fragrance without unduly exposing the user to liquid leakage immediately after capsule breakage. While not shown, the absorbent medium **14** may be fabricated with axial or radial channels to facilitate diffusion of the fragrance compound(s) toward the exterior environment. In another embodiment, the fragrance delivery device **10** is void of any perforations in the outer paper covering **16.**

The dried, breakable fragrance-loaded capsules **12** are combined with the absorbent medium **14,** wrapped in an outer paper covering **16** to form the fragrance delivery device **10,** using one or two strips of adhesive (e.g., hot melt adhesive or polyvinyl alcohol adhesive) to anchor the absorbent medium **14** to the outer paper covering **16,** and a single line of adhesive to glue the seam of the outer paper covering **16** thereby forming a single outer surface of the device **10.** The fragrance delivery device **10** may be manufactured using standard or modified cigarette filter manufacturing machinery with capsule inserter. For example, machinery manufactured by ITM group (e.g. ITM Poland or ITM Solaris) or Hauni group (e.g., FLEXPORT CI module) may be used to prepare the fragrance delivery device **10** described herein.

In accordance with yet another embodiment of the present invention, a method of using the fragrance delivery device **10** is provided. The method includes providing the fragrance delivery device **10** to a person; detecting, decoding, and/or transmitting the two dimensional bar code by a visual detection application residing on a portable electronic device **38** to a remote server **40;** transmitting information from the remote server **38** relating to the fragrance delivery device **10** and receiving said information at the portable electronic device **38;** and displaying said information on the portable electronic device **38.** The method may be performed prior to, after, or concurrent with breaking a fragrance-loaded seamless capsule **12** and the subsequent olfactory experience. Advantageously, the user may have direct access to information related to the fragrance, such as its composition, ingredient origin, supplier information, GREEN MOTION^{™} index, and other details likely relevant to the user, such as ratings or comments from other users or inspirational comments from the creating perfumer. In another embodiment, the method further includes creating a file to store personal or product related information, and that file may be stored on the portable electronic device **38** or on the remote server **40.** In accordance with embodiments of the present invention, the method further comprises rupturing the capsule shell **22** by application of a sufficient crushing force to release the liquid core **24,** wherein the adjacent absorbent medium **14** absorbs the liquid core **24** thereby gradually transporting the fragrance compounds in few seconds toward the outer paper covering **16** and then diffusing into the adjacent surroundings to allow the person to experience the fragrance compound.

The invention is hereunder illustrated by the following examples, which should not be considered as limiting the scope of the invention.

### EXAMPLES

*General procedure for preparing external aqueous phase:* A measured quantity of process water is heated and a hydrocolloid gelling agent mixed therein until complete dissolution is achieved. Filler(s), crosslinking agents, and/or other additives, are added and the resultant mixture is stirred until it is coextruded to make the breakable seamless capsule. Exemplary formulations for preparing the external aqueous phase of gellable mixtures are shown in Tables 1 and 2.

**Table 1: Gellable Mixture Composition A.**

| **Ingredient** | **Amount** |
|---|---|
| SORBITOL | 1.50% |
| CITRIC ACID | 0.20% |
| CARRAGEENAN | 4.00% |
| SODIUM HYDROXIDE 50% | 0.18% |
| GELLAN GUM | 1.50% |
| PROCESS WATER | 92.62% |

**Table 2: Gellable Mixture Composition B.**

| **Ingredient** | **Amount** |
|---|---|
| SORBITOL | 1.00% |
| GLYCERINE | 0.50% |
| SODIUM CITRATE | 0.10% |
| GELLAN GUM | 1.70% |
| PULLULAN GUM | 1.50% |
| CARRAGEENAN | 0.80% |
| DEXTRIN | 6.00% |
| MODIFIED STARCH | 1.00% |
| POTASSIUM CHLORIDE | 0.12% |
| PROCESS WATER | 87.28% |

*General procedure for preparing internal oily phase liquid core:* A desired quantity of fragrance ingredients are mixed with a measured quantity of the non-glyceride solvent. In some instances a small quantity (<10 wt%) of ethanol may be used to homogenize the liquid core. The final liquid core formulation should be a stable liquid at room temperature. Exemplary formulations for various liquid core formulations comprising fragrance, isopropyl myristate (IPM) solvent, and ethanol are shown in Table 3.

**Table 3: Liquid Core formulations.**

| **Example** | **Fragrance** | **Amount** | **IPM Solvent** | **Ethanol** |
|---|---|---|---|---|
| Core 1 | Fragrance A | **50%** | **47%** | **3%** |
| Core 2 | Fragrance A | **20%** | **77%** | **3%** |
| Core 3 | Fragrance A | **80%** | 17% | **3%** |
| Core 4 | Fragrance B | **50%** | **47%** | **3%** |
| Core 5 | Fragrance C | **50%** | **47%** | **3%** |
| Core 6 | Fragrance D | **50%** | **47%** | **3%** |
| Core 7 | Geranium essential oil | **50%** | **47%** | **3%** |
| Core 8 | Geranium essential oil | **20%** | **77%** | **3%** |
| Core 9 | Geranium essential oil | **80%** | **17%** | **3%** |
| Core 10 | Patchouli essential oil | **50%** | **47%** | **3%** |

*General procedure for preparing capsules:* The external aqueous phase of the gellable shell mixture and the internal oily phase of the liquid core are individually pumped through a submerged coaxial nozzle assembly thereby forming a concentric composite stream that partitions into discreet concentric drops due to the vibrational energy imparted thereto. The discharge of the coaxial nozzle is submerged into a carrier fluid (e.g., a medium chain triglyceride (MCT)), which is at a temperature lower than the gelation temperature of the gellable mixture. The gellable shell mixture is thereby cooled and forms the hydrated shell portion of the capsule. The capsules thus formed are then aged at 4°C for about an hour, collected and centrifuged to remove a majority of the residual MCT. The centrifuged capsules and a portion of desiccating agent (e.g., silica or starch) are mixed and then dried with air at 35 °C until a desired reduction in water is achieved in the dried capsules (< 5 wt% water), which are then collected and sieved.

The dried, breakable fragrance-loaded capsules prepared in accordance with embodiments of the present invention have a homogeneous and smooth appearance, and are spherical or substantially spherical (i.e., shape ratio >0.9), and are characterized the desired hardness (Cᵢ) and elasticity (see Table 4).

**Table 4: Fragrance-loaded seamless capsule characteristics.**

| Capsule | Shell | Core | Size (mm) | Shape Ratio | Hardness (Ci) (kg) | Elasticity (%) |
|---|---|---|---|---|---|---|
| 1 | A | Core 1 | 3.60 +/-0.08 | 0.98 +/-0.01 | 1.04 +/-0.89 | 45.1% +/-1.6% |
| 2 | A | Core 4 | 3.60 +/-0.06 | 0.98 +/-0.01 | 1.38 +/-0.85 | 42.6% +/-1.5% |
| 3 | A | Core 5 | 3.60 +/-0.05 | 0.97 +/-0.01 | 0.96 +/-0.19 | 34.9% +/-3.5% |
| 4 | B | Core 1 | 3.57 +/-0.04 | 0.97 +/-0.01 | 1.27 +/-0.19 | 31.1% +/-5.1% |
| 5 | B | Core 6 | 3.52 +/-0.04 | 0.97 +/-0.01 | 1.41 +/-0.09 | 27.4% +/-3.9% |
| 6 | B | Core 7 | 3.55 +/-0.04 | 0.97 +/-0.01 | 1.49 +/-0.18 | 36.7% +/-3.8% |
| 7 | B | Core 8 | 3.56 +/-0.03 | 0.98 +/-0.01 | 1.47 +/-0.24 | 33.3% +/-4.7% |
| 8 | B | Core 9 | 3.56 +/-0.15 | 0.97 +/-0.01 | 1.26 +/-0.24 | 29.4% +/-5.1% |
| 9 | B | Core 10 | 3.54 +/-0.03 | 0.97 +/-0.01 | 1.54 +/-0.18 | 41.2% +/-5.9% |

The dried, breakable fragrance-loaded capsules **12** were combined with the absorbent medium **14,** wrapped in an outer paper covering **16** to form the fragrance delivery device **10** and were manufactured using standard or modified filter making techniques and equipment, with capsule inserter.

**Table 5: Fragrance Delivery Device Characteristics.**

| | **Inventive Device 1** | **Inventive Device 2** | **Inventive Device 3** | **Inventive Device 4** | **Inventive Device 5** |
|---|---|---|---|---|---|
| LENGTH (mm) | 150 | 150 | 150 | 150 | 150 |
| DIAMETER (mm) | 5.7 | 5.7 | 5.7 | 5.7 | 5.7 |
| CAPSULE (mm) | 3.5 | 3.5 | 3.5 | 3.5 | 3.5 |
| FINISHED WEIGHT (mg) | 717 | 620 | 780 | 730 | 800 |

**Table 6: Comparison of inventive fragrance delivery device against traditional perfumer blotter strip.**

| **Example** | **Form** | **Fragrance** |
|---|---|---|
| Inventive Device 1 | Cylindrical, 150 mm x 5.7 mm | Core 1 |
| Comparative 2 | Blotter, 150 mm x 7 mm/ (LxW) | Fragrance A in Ethanol (20 wt%) |

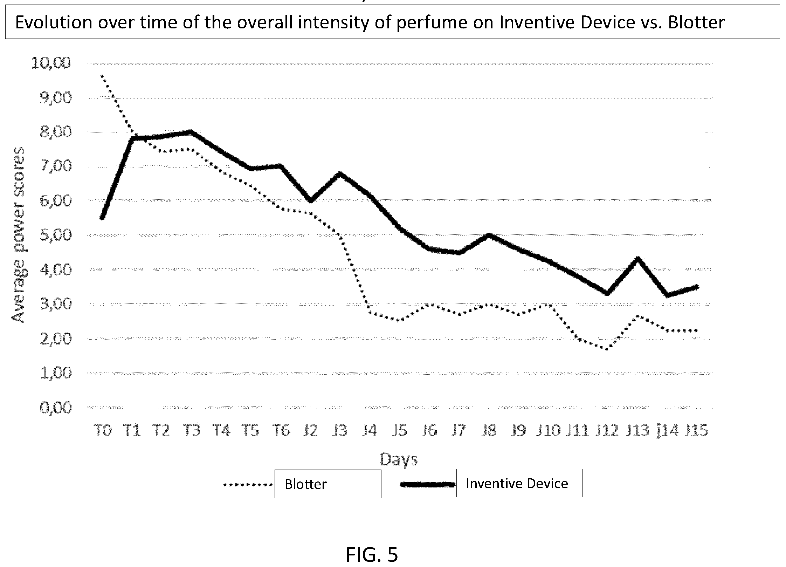

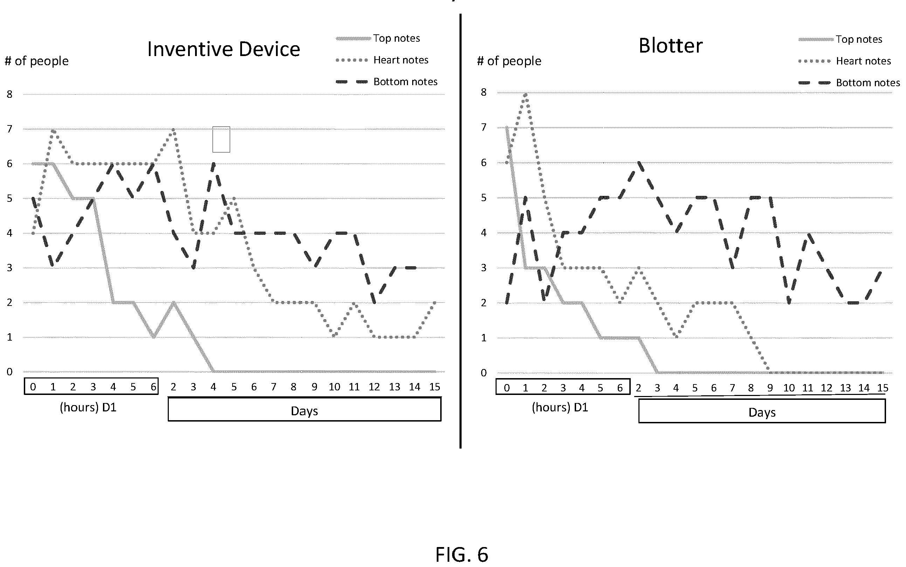

*Sensory Testing and Comparison:* As shown in FIGS. 5 and 6, a traditional perfumer fragrance blotter strip (150 mm x 7 mm) dosed with a measured quantity of perfume composition similar to the quantity of the capsule core (22 µL) was compared against the inventive fragrance delivery devices **10** manufactured in devices 1 or 2, in Table 6. Eight panelists tested inventive fragrance delivery devices against the traditional blotter every day over a 15 day period, except weekends. Evaluations of fragrance A were made regarding top notes, heart notes, and bottom notes (FIG. 6), and overall fragrance intensity (FIG. 5) at specified time intervals (0 h, 2 h, 3 h, 4 h, 5 h, 6 h, and 2 - 15 days) and graded on a scale of 0-10. While the traditional blotter provided a more intense sensory experience in the first two hours, the inventive fragrance delivery device **10** proved to be substantially longer lasting even days later. Moreover, the inventive fragrance delivery device provided better and longer lasting diffusion of the top and heart notes vis-à-vis the traditional blotter. Furthermore, the inventive fragrance delivery device **10** allows the user to smell at the same time the top and bottom notes especially during the first day of the evaluation. Advantageously, the inventive fragrance delivery device **10** also comprises a plurality of fragrance-loaded seamless capsules, which provides the added advantage of breaking another capsule even weeks later.

These sensory panel results were confirmed by HS-GC/MS analysis showing that there is quantitatively more fragrance remaining on the fragrance delivery device of the invention after 24 h and 48 h, than on a traditional blotter. The evaporation of the fragrance on an inventive device and a standard blotter was studied by measuring the residual fragrance A over time. For this, a 7890 Gas Chromatograph (Agilent) equipped with 2 injectors and 2 detectors (FID and MS detector 5977 MSD Agilent) was used. The analytical system was driven using ChemStation software (Agilent, version E01.00.237). Pieces of 2 cm around each capsule of the inventive device were cut. Pieces of 2 cm of blotters were also prepared. At T₀: capsules of the inventive device were cracked and 22 µL of fragrance were deposited with a micropipette on pieces of blotters. Fragrance deposited on blotters was the same as core capsule (fragrance A in 50% of isopropyl myristate) and 22 µL represents the volume of one capsule. The extractions of the residual fragrance on each device were carried out after different evaporation times by adding accurately an internal standard (20 mg of a methyl octanoate solution at 30% in heptane) and 10 mL of dichloromethane. Samples were stirred for 30 min on a shaking table at 300 rpm. Organic phases were filtered on PS and injected in GC-MS for identification and quantification. The quantitative approach uses FID integration data. Compliance with the ISO11024 standard has been verified beforehand. The internal calibration curve is determined with four reference solutions containing different ratios of fragrance and internal standard in 10 mL of dichloromethane. The fragrance quantified at T₀ is defined as the quantity of fragrance available for each device.

While the invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative product and/or method and examples shown and described. The various features of exemplary embodiments described herein may be used in any combination. Accordingly, departures may be made from such details without departing from the scope of the general inventive concept.

## Claims

1. A fragrance delivery device, comprising, consisting essentially of, or consisting of:
a plurality of fragrance-loaded seamless capsules, each having a capsule shell surrounding a liquid core containing a fragrance compound and a non-glyceride lipophilic solvent, wherein the capsule shell comprises a hydrocolloid gelling agent and a filler material, and the liquid core having a relative density between 0.85 and 0.99;
an absorbent medium embodying the plurality of fragrance-loaded seamless capsules along an axial direction in both directions; and
an outer paper covering surrounding and containing the plurality of fragrance-loaded seamless capsules and the absorbent medium, wherein the covering is adapted to allow the fragrance compound to diffuse therethrough, and wherein the outer paper covering comprises a printed indicia, for example including a one- or two-dimensional barcode;
wherein each fragrance-loaded seamless capsule is breakable having an initial crush strength (Cᵢ) from 0.5 to 2.5 kg, elasticity up to 60%, and a shape ratio equal to 0.9 or higher; wherein upon rupturing the capsule shell by application of a sufficient crushing force the liquid core is released and absorbed into the adjacent absorbent medium thereby gradually dispersing the fragrance compound toward the outer paper covering.

2. The fragrance delivery device according to claim 1, wherein the non-glyceride lipophilic solvent is miscible with ethanol and has:
a viscosity of less than 10 mPa•s as measured at a temperature of 25 °C and a shear rate of 10 s⁻¹;
a relative density of between 0.85 and 0.99; and
a spread value greater than 850 mm² / 10 min.

3. The fragrance delivery device according to claim 2, wherein the non-glyceride lipophilic solvent is selected from the group consisting of non-glycerol esters, silicones, vegetable oils, and mixtures thereof.

4. The fragrance delivery device according to claim 2, wherein the non-glyceride lipophilic solvent is selected from the group consisting of isopropyl myristate, isoadipate, cococaprylate, polydimethylsiloxane, octamethyltrisiloxane, dimethicones, grapeseed oil, and mixtures thereof.

5. The fragrance delivery device according to any preceding claim 1 to 4, said fragrance delivery device having a substantially cylindrical or cylindrical body comprising a distal end and a proximal end, along with the plurality of the fragrance-loaded seamless capsules aligned along the axial direction of a central axis of the cylindrical body; wherein the plurality of capsules are spaced apart and separated by portions of the absorbent medium.

6. The fragrance delivery device according to any preceding claim 1 to 5, wherein outer paper covering comprises, consists essentially of, or consists of, a coated paper having a density between 22 gsm and 90 gsm, and **characterized by** a porosity of less than 5 CU.

7. The fragrance delivery device according to any preceding claim 1 to 6, wherein the outer paper covering further comprises capsule location identifiers, such as capsule location markings, indicating the location of the fragrance-loaded seamless capsules.

8. A fragrance delivery system comprising, consisting essentially of, or consisting of, the fragrance delivery device according to any preceding claim 1 to 7, and a remote server linked to the printed indicia, said remote server being adapted to transmit and display information relating to the fragrance delivery device to/on a portable electronic device upon detection, decoding, and/or transmission of printed indicia, for example of the one-or two-dimensional bar code, by a visual detection application residing on the portable electronic device.

9. The fragrance delivery system according to claim 8, wherein a user of the portable electronic device and the fragrance delivery device is provided a file to store personal or product related information, said file being stored on the portable electronic device or on the remote server.

10. Use of the fragrance delivery device according to any preceding claim 1 to 7 or the fragrance delivery system of claim 8 or claim 9, for allowing a person to experience the fragrance compound.

11. Use of the fragrance delivery device according to any preceding claim 1 to 7 or the fragrance delivery system of claim 8 or claim 9, for sampling and/or marketing the fragrance compound.

12. Method of using the fragrance delivery system of claim 8 or claim 9, said method comprising:
providing the fragrance delivery device to a person;
detecting, decoding, and transmitting the printed indicia, for example the one- or two-dimensional bar code, by a visual detection application residing on a portable electronic device to a remote server;
transmitting information relating to the fragrance delivery device from the remote server to the portable electronic device; and
displaying said information on the portable electronic device.

13. Method according to claim 12, further comprising:
creating a file to store personal or product related information, said file being stored on the portable electronic device or on the remote server.

14. Method according to claim 12 or 13, further comprising:
rupturing the capsule shell by application of a sufficient crushing force to release the liquid core, wherein the adjacent absorbent medium absorbs the liquid core thereby gradually transporting the fragrance compounds, preferably in few seconds, toward the outer paper covering and then diffusing into the adjacent surroundings to allow the person to experience the fragrance compound.
